# EUROPEAN PATENT APPLICATION

(11) **EP 4 796 032 A1**
(43) Date of publication of application: **26.08.2026**
(21) Application number: 25382160.7
(22) Date of filing: 20.02.2025
(51) Int. Cl.: A61B 5/1455, A61B 5/16, A61B 5/00

(54) **COMPUTER IMPLEMENTED METHOD FOR SELECTING EMOTIONAL STIMULI CONTROL AMONG EMOTIONAL STIMULI CANDIDATES TO BE USED IN SIGNAL DENOISING**

(71) Applicant: Newmanbrain, S.L., 03202 Elche (ES)
(72) Inventor: IBAÑEZ BALLESTEROS, Joaquín, 03540 ALICANTE (ES); MOLINA RODRIGUEZ, Sergio, 03181 TORREVIEJA (ES); BELMONTE MARTINEZ, Carlos, 03550 SAN JUAN DE ALICANTE (ES)
(74) Representative: Sugrañes, S.L.P.

(57) **Abstract**

Computer implemented method for selecting emotional stimuli control (X) among emotional stimuli candidates (V,S,N) for a group of individuals to be used in signal denoising to isolate the true cerebral signal, wherein the method comprises the following sequence of steps: determining fNIRS related superficial signals (LS,RS) and neural signals (NS) for each of the individual of the group subject to emotional stimuli candidates; calculating a superficial signal group response (SGR) and a neural signal group response (NGR) for each emotional stimuli candidate, and the significance (A,B) of each group responses; and selecting as an emotional stimuli control (X) for the group an emotional stimuli that does have a significance (A) superficial signal group response (SGR), and has a no-significance (B) corresponding neural signal group response (NGR). Also a data processing apparatus, a computer program and a computer-readable storage medium, related to the computer implemented method are disclosed.

## Description

### Technical field of the invention

The invention relates to a computer implemented method for selecting emotional stimuli control to be used in signal denoising methods. The invention also relates to a data processing apparatus, a computer program and a computer-readable medium comprising instructions related to the computer implemented method.

### Background of the invention

Emotional responses are fundamental to human functioning, but involve multiple cognitive functions that interact across different brain regions (Morawetz et al., 2017), making it challenging to disentangle the underlying neural mechanisms (Saarimäki, 2021). Neuroimaging studies attempt to relate emotions to sensor data and from them identify relevant features of neural processing. Among other methods, such as the generalized linear model (GLM), inter-subject correlation (ISC) analysis has been proposed to extract features based on measuring the consistency of neural responses across individuals (for review, see Nastase et al., 2019).

Firstly introduced by Hasson et al. (2004), ISC analysis is a widely used data-driven approach for detecting synchronized brain activity across a group of participants exposed to common complex stimuli. When individuals' brains process the same sequence of stimuli, only the brain areas involved would respond consistently across subjects, which can be assessed using fairly simple measures of similarity, such as correlation (Nastase et al., 2019). Since ISC analysis is based simply on capturing the temporal synchronization between subjects' responses, there is no need to specify any priori model as in the traditional GLM framework. This "model-free" property of ISC analysis makes it well suited when it is challenging to generate a hypothesis about the expected response to the stimulus (Adolphs et al., 2016). It should be noted that, although it is typically applied to study brain activity evoked by naturalistic stimuli (such as movies), ISC-based analysis can also be used with block-design tasks, provided that the timing of the stimuli is the same for all subjects (Bordier & Macaluso, 2015; Hejnar et al., 2007; Pajula et al., 2012).

fMRI studies have demonstrated that consistent neural responses, as measured by ISC, could be evoked by naturalistic paradigms (Hasson et al., 2004, 2010). However, some brain regions tend to be driven more consistently by external stimuli than others, which have led to define "extrinsic" systems showing higher ISC reliability (e.g. sensory-motor areas) and "intrinsic" systems that are engaged with lower consistency (e.g. prefrontal and cingulate cortex) (Golland et al., 2007; Hasson et al., 2010; Ren et al., 2017). Beyond this simple binary division, a hierarchical arrangement has been proposed to better explain the decrease in ISC levels as information processing progress from primary sensory cortices to higher-order brain regions (Ren et al., 2017). In this sense, it is believed that inter-subject variability of internal mental processes leads to more idiosyncratic and thus less common responses to external stimuli, which is reflected in lower levels of ISC (Hasson et al., 2010; Ren et al., 2017).

It is known that qualitatively different stimuli can elicit responses that vary both in consistency and in the brain areas involved (Nastase et al., 2019), which would allow emotions to be classified based on identifiable activity patterns (Jääskeläinen et al., 2021). A stimulus material widely used in neuroimaging studies are scenes from the International Affective Picture System (IAPS) (Bradley & Lang, 2007), which appear effective to consistently engage certain brain regions (Morawetz et al., 2017). Regardless of the type of emotional stimulus, the prefrontal cortex (PFC) has been repeatedly highlighted in emotion research (for review, see Dixon et al., 2017), with particular focus on the role of the ventromedial PFC (VMPFC) in the regulation of negative emotions (for review, see Hiser & Koenigs, 2018).

To advance emotion research, friendlier experimental settings using non-intrusive sensors could be especially useful. On this line, functional near-infrared spectroscopy (fNIRS) is a promising neuroimaging tool. fNIRS is an optical neuroimaging technique for monitoring hemodynamic responses related to brain activity (Scholkmann et al., 2014) that, among several other applications (Pinti et al., 2018), shows great potential in emotional research (Bendall et al., 2016; Doi et al., 2013). fNIRS is comparatively affordable, easy to use and imposes less physical and psychological discomfort than other techniques such as fMRI (Tuscan et al., 2013). However, compared to fMRI and other neuroimaging modalities, less research has been done on ISC using fNIRS. Studies on social interactions by hyperscanning (for review, see Nam et al., 2020), music listening (Da Silva Ferreira Barreto et al., 2020) or narratives (Rowland et al., 2018), and movies viewing (Somech et al., 2022) are some notable exceptions.

Despite its advantages, fNIRS is not suitable for probing deep brain regions, as the penetration of infrared light through tissue is mostly limited to the superficial cortex (Strangman et al., 2013). Fortunately, the most rostral PFC can be easily explored by placing the NIRS probe on the hairless skin of the forehead. However, fNIRS studies have shown mixed results in relating hemodynamic changes to emotional experience, perception or regulation, which has raised concerns about the lack of standardization in experimental designs (for review, see Westgarth et al., 2021).

Differences in instrumentation and signal processing contribute to this problem. For example, overlook the well-known drawback of fNIRS that relates to confounding components not originating in the cerebral cortex, such as local blood flow changes in surface tissues (e.g. skin, muscle, skull) and systemic activities (e.g. cardiovascular, respiratory) (Kirilina et al., 2012; Saager et al., 2011; Tachtsidis & Scholkmann, 2016). In particular, emotional stimuli can cause consistent local and systemic hemodynamic fluctuations that can lead to misinterpretations (Simony & Chang, 2020). For example, facial vasomotion is part of emotional expression in humans (Salazar-López et al., 2015) and consistent responses have been found among subjects using thermal imaging under emotional induction (Sonkusare et al., 2020).

With respect to the temporal scales of neural processing, a hierarchy of temporal receptive windows has been suggested in which higher-order areas require coherent unfolding of the stimulus over longer time scales to show coherent information processing (Hasson et al., 2008; Jääskeläinen et al., 2008; Lerner et al., 2011). In particular, emotional stimuli can evoke slowly evolving and temporally varying responses across subjects, which implies identifying relevant frequency ranges to capture consistent ISC, typically bellow 0.1 Hz (Nastase et al., 2019). In support of this, stronger ISCs at low frequencies have been found in hierarchically higher cortical areas in electrocorticography (Honey et al., 2012) and fMRI (Kauppi et al., 2010) studies. In this sense, since it provides better temporal resolution than fMRI, fNIRS would allow a finer frequency decomposition of hemodynamic responses and thus a more precise time-scale localisation of the shared fluctuations.

Therefore, it is an objective of the present invention to obtain a computer implemented method that can be used to determine an emotional stimuli that could be used to check the reliability of NIRS signal denoising methods, and more precisely fNIRS signal denoising methods.

### Description of the invention

The invention relates to a computer implemented method for selecting emotional stimuli control among emotional stimuli candidates for a group of individuals, participants, wherein the method comprises the following sequence of steps: determining fNIRS related superficial signals and neural signals for each of the individual of the group subject to emotional stimuli candidates; calculating a superficial signal group response and a neural signal group response for each emotional stimuli candidate, and the significance of each group responses; and selecting as an emotional stimuli control for the group an emotional stimuli that does have a significance superficial signal group response, and has a no-significance neural signal group response.

Advantageously, this emotional stimuli control has an identifiable effect on surface activity that can be used to check the reliability of signal denoising methods, for example can be used in signal denoising to isolate the true cerebral signal. Moreover, it also satisfies the need for controlling surface interference, especially when using emotional stimulation. The group of individuals, also known as participants, are expected to share at least a characteristic among them, so the emotional stimuli control, once selected, can be used for processing fNIRS signals from future participants that would share the at least one characteristic with the group. Groups can be formed sharing one or more characteristics, such as gender, age range, mental or brain disorder conditions, body physiological conditions or combination. For example, the mental or brain disorder condition may be one of: attention-deficit/hyperactivity disorder, mild-cognitive impairment, neurodegenerative disorders as Alzheimer's disease, dysautonomia disorders, depression, or anxiety. However, other conditions may also be envisaged.

It is expected that the method described could be programmed and executed by a computer, for example a local, remote, distributed computer or an embedded device. Further, a program including instructions that when executed cause a machine to perform the methods described are envisaged. The fNIRS signals to be processed by the computer implemented method can be obtained directly from the participants in real-time, or be obtained from a repository where the signals have been previously stored.

In an embodiment the step of determining fNIRS related superficial signals and neural signals or each of the individual of the group subject to each emotional stimuli candidate comprises: obtaining fNIRS long superficial signals and short superficial signals; and calculating a corresponding neural signal and reference superficial signal from the long superficial signals and the short superficial signals. Therefore, from fNIRS signals obtained from the brain, signals for calculating a superficial signal group response and a neural signal group response can be obtained.

In an embodiment, emotional stimuli candidates are associated with threshold levels of valence and arousal. So, each emotional stimuli candidate can be defined by a cluster within levels of valence and arousal. It is however contemplated that clusters of stimuli candidates may also partly or completely overlap.

In an embodiment, the step of determining fNIRS superficial signal and neural signals for each of the individual of the group subject to each emotional stimuli candidate comprises subjecting each individual to a sequence of emotional stimuli candidates. Preferably, the sequence should alternate emotional stimuli candidates and also should provide a neutral emotional stimuli between each pair of emotional stimuli candidates, avoiding the reaction from the participant from an emotional stimuli candidate to propagate to the next. Also, a relaxing o blank time, long enough to avoid interference of reactions could be used between emotional stimuli candidates.

In an embodiment, in that emotional stimuli candidates are represented by pictures to be seen by the individuals, also known as participants. Naturally, other representations of the emotional stimuli candidates such as auditive (for example songs or voices) or even tactile stimuli are also envisaged.

In an embodiment, superficial signals and neural signals are associated with a chromophore, such as HbO or HbR, a channel of the signal on the brain, and an oscillatory component of the signals. Therefore, the fNIRS signals can be representative of a HbO or HbR chromophore, or the place where it has been obtained from the brain, channel, and an oscillatory component of the signals, so only a range of frequencies are kept in the signal. For example, superficial signals and neural signals can be obtained from emitters and receptors placed in different parts of the skin of an individual, for example in different parts of the skin of the forehead, combinations of emitters and receptors determining channels.

In an embodiment, the significance of the group response for each emotional stimuli candidate is calculated using significance threshold of an intersubject correlation, ISC.

In an embodiment, the step of calculating a superficial signal group response and a neural signal group response subject to each emotional stimuli candidate comprises a multiresolution analysis (MRA), preferably wavelet based.

In an embodiment, the multiresolution analysis comprises an undecimated maximal overlap discrete transform (MODWT), also known as the stationary wavelet transform (SWT). Other techniques for multiresolution analysis are, for example, Empirical Mode Decomposition (EMD), Variational Mode Decomposition (VMD) and Empirical Wavelet Transform (EWT).

In an embodiment, the method further comprises obtaining a signal time-domain reconstruction at each decomposition level to isolate different oscillatory components.

In an embodiment, the signal time-domain reconstruction comprises separating a signal into oscillatory components. For example, it could be separated into nine oscillatory components, comprising different bandwidths, but other numbers of oscillatory components are also envisaged.

The invention further comprises a data processing apparatus comprising a processor configured to perform the steps of the disclosed computer implemented method.

The invention further comprises a computer program comprising instructions which, when the program is executed by a computer, cause the computer to carry out the steps of the disclosed computer implemented method.

The invention comprises a computer-readable medium comprising instructions which, when executed by a computer, cause the computer to carry out the steps of the disclosed computer implemented the computer implemented disclosed method.

### Brief description of the drawings

As a complement to the description provided herein and for the purpose of helping to make the characteristics of the invention more readily understandable, this specification is accompanied by a set of drawings, which by the way of illustration and not limitation, represent the following:
Fig. 1 presents clusters of emotional stimuli candidates;
Fig. 2 presents a schematic diagram of the experimental procedure;
Figs. 3a and 3b present a schematic illustration of the channels between emitters and receivers, and their placement over the brain;
Fig. 4 represents a schematic diagram of the MRA procedure; and
Fig. 5 represents the group average of the z-scored time courses of HbO and HbR chromophores of channels, and ISC of HbO chromophore, for a series of emotional stimuli candidates for oscillatory component C9.

### Detailed description of an embodiment of the invention

It is intended to detect whether one or more emotional stimuli S,V,N triggered by affective pictures evoke frequency-specific hemodynamic patterns, capturable by ISC-based analysis from prefrontal fNIRS recordings that could be used in signal denoising to isolate the true cerebral signal by determining fNIRS related superficial signals LS,RS and neural signals NS for individual of a group subject to emotional stimuli candidates; and by then calculating a superficial signal group response SGR and a neural signal group response NGR for each emotional stimuli candidate, and the significance A,B of each group responses. Then the emotional stimuli control X for the group can be selected as the emotional stimuli that does have a significance A superficial signal group response SGR, and has a corresponding no-significance B neural signal group response NGR.

Given the known influence of gender on emotional experience (Stevens & Hamann, 2012; Whittle et al., 2011) and that the similarity in brain activity and behaviour seems to be greater in women (Finn et al., 2017), we constrained this study to women only. As stimuli we used two types of highly arousing IAPS scenes, namely: low-valence violence pictures based on the extensive use of negative stimuli in research (Huang et al., 2017) and their particular influence on women's PFC activity (Faraone et al., 2021; Glotzbach et al., 2011; Stevens & Hamann, 2012), and (2) high-valence sexual imagery because it can also elicit distinguishable autonomic responses (Bermond et al., 2010; DePesa & Cassisi, 2017; Ritz et al., 2005).

It is hypothesized that a fixed sequence of pictures, grouped by similar valence and arousal, would induce emotional states long-lasting enough to allow the identification of common responses on different time-scales. Second, we expected higher ISC values in particular frequency bands. Third, we further expected the responses to be related to emotional content. Fourth, emotional stimuli would also evoke common changes in extra-cerebral fNIRS signals. To verify these hypotheses, we present an experimental design that combines: (1) fixed blocks of affective pictures for emotion induction, (2) multiresolution analysis to identify the relevant temporal scales (i.e., frequency-bands), and (3) a NIRS device that allows multi-distance measurements to improve the separation of cerebral and extracerebral components.

Hereinafter, an embodiment of the method will be explained in detail.

### Participants

The initial sample was composed by twenty-three women who were recruited from different university faculties. Prior to participation, the aim of this research was communicated and the doubts about the experimental protocol were solved. All participants signed a written consent approved by the Ethical Committee of the university in accordance with the Declarations of Helsinki (Ref: DF.JIB.01.15). Only females were selected to avoid gender influences (Stevens & Hamann, 2012) and because their response similarity is usually higher (Finn et al., 2017). All participants were right-handed, with normal or corrected-to-normal vision, with no known psychiatric or neurological disorders, and self-identified as heterosexual. Three participants were discarded due to signal quality issues, as we will explain later (see section 2.5). Therefore, the final sample consisted of twenty women (M age= 21.25; SD =2.98; range = 18-28).

### Emotional stimuli

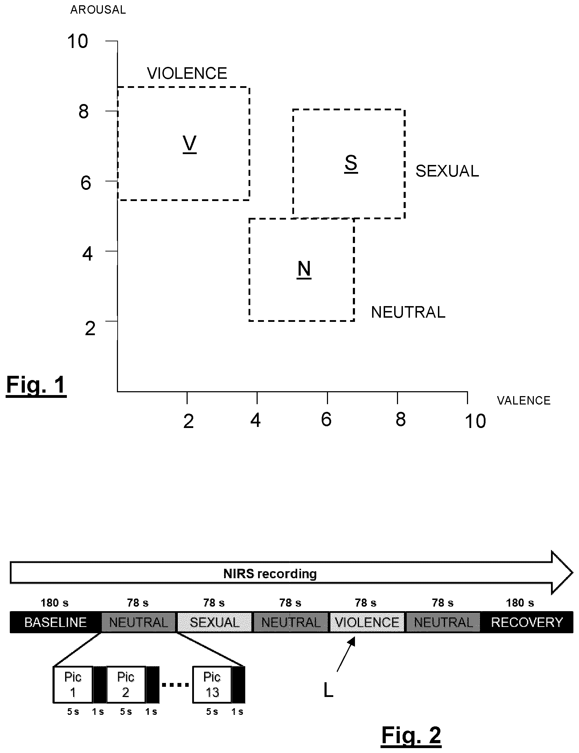

In this work, we employed a set of pictures extracted from the International Affective Picture System (IAPS) (Bradley & Lang, 2007; Moltó et al., 2013), a tool widely used in affective research. Therefore, emotional stimuli candidates (V,S,N) are represented by pictures Pic to be seen by the individuals. All the selected pictures contained people with their faces clearly visible to enhance the emotional response (Groen et al., 2013). For each category, the pictures were selected on the basis of normative valence and arousal, all of them depicting social interactions, and adapted to our country population (Moltó et al., 2013), namely: neutral/none, sexual (nude hetero couples) and violence. (Table 1). For sexual and violence content, the selected pictures shared similar high arousal, but with positive and negative valence respectively (Table 1). The neutral pictures were located around the middle of the valence scale and with low arousal. Therefore, emotional stimuli candidates V,S,N are associated with threshold levels of valence and arousal, for example, neutral N can be considered to be clustered with valence between 4 and 7 threshold and arousal between 2 and 5 threshold, sexual S can be considered to be clustered with valence between 5 and 8,5 threshold and arousal between 5 and 8 threshold, violence V can be considered to be clustered with valence between 0 and 4 threshold and arousal between 8,5 threshold and 5,5 threshold. The pictures, and therefore the emotional stimuli candidates V,S,N can be clustered as presented in Fig. 1.

### Procedure

The experiment took place in the morning (between 12:00 pm and 13:00 pm). The presentation of the pictures was controlled by a custom application that ran on a PC and was fully synchronized with the recording system. The pictures were displayed on a 21" LCD monitor placed at 80 cm of the participant, who was sitting in an ergonomic chair in a dimly lit room and at a comfortable temperature.

As presented in Fig. 2, emotion induction was realized by requiring participants to view a fixed sequence L of picture Pic. During the viewing, participants were asked to qualitatively label each picture Pic simply by using two buttons on a response pad: the right button for pleasant, the left one for unpleasant and none for neutral/indifferent. Thus, they were not limited to passive viewing but engaged in basic emotional judgement, which also warranted attention. The experiment began with a 180 s baseline recording and ended with another 180-s recovery period, during which participants were asked to remain as relaxed as possible while viewing a blank screen. After baseline, the five picture blocks were presented in a fixed order: neutral-sexual-neutral-violence-neutral. Every block contained 13 pictures Pic, each displayed for 5-s followed by a black screen for 1 s to reduce visual persistence. Thus, each block lasted (5 + 1) * 13 = 78 s, within which the pictures Pic were presented in exactly the same order to all the individuals participants of the group. Note that the sexual and violence blocks were placed between neutral blocks, which are long enough to wash-out possible persistence effects. Immediately after recording, participants were required to rate the pictures on a Self-Assessment Manikin (SAM) nine-points scale (Bradley & Lang, 1994). Ratings were analysed by repeated measures ANOVA for arousal and valence with the factor "picture category". Post-hoc contrasts were computed for differences. Therefore, the step of determining fNIRS superficial signal (LS,RS) and neural signals (NS) for each of the individual of the group subject to each emotional stimuli candidate (V,S,N) comprises subjecting each individual to a sequence (L) of emotional stimuli candidates.

**Table 1. Normative arousal and valence of selected images.**

| Selected images | Valence M (SD) | Arousal M (SD) |
|---|---|---|
| Neutral (2036, 2102, 2191, 2235 ,2384, 2393, 2396, 2397, 2411, 2579, 2593, 2850, 7550) | 5.55 (0.51) | 3.51 (0.47) |
| Sexual (4290,4647, 4649, 4652, 4658, 4664.1, 4670, 4672, 4680 ,4681,4690, 4800, 4810) | 6.80 (0.55) | 6.73 (0.55) |
| Violence (3500, 3530, 6212, 6312, 6315, 6520, 6560, 6571, 6834, 9252, 9413, 9414, 9427) | 2.07 (0.59) | 7.01(0.48) |

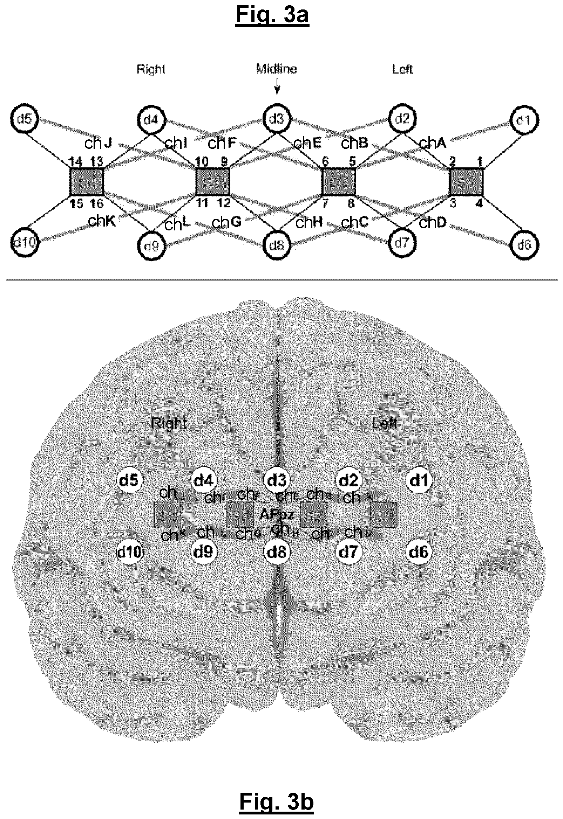

A newly developed NIRS device (Tehia, Newmanbrain, S.L., Elche, Spain), equipped with 16 short-channels (14 mm) and 12 long-channels (32 mm), which was introduced in a previous study (Molina-Rodriguez et al., 2022). Briefly, it is a multichannel continuous-wave NIRS device, providing multiple channels chA,chB,chC,chD,chE,chF,chG,chH,chl,chJ,chK,chL, equipped with four emitters s1,s2,s3,s4 and ten photo detectors d1,d2,d3,d4,d5,d6,d7,d8,d9,d10, receivers, arranged in a rectangular 80x20 mm patch. Each emitter s1,s2,s3,s4 housed two light-emitting-diodes (LED) at wavelengths 740 nm and 850 nm. Through its duty cycle, the device combines emitters s1,s2,s3,s4 and detectors d1,d2,d3,d4,d5,d6,d7,d8,d9,d10 at two separation distances to provide 16 short-channels (14 mm) and 12 long-channels (32 mm). Additionally, it corrects for ambient light interference and records motion activity by a 3-axis accelerometer. Data is transferred via Bluetooth at a sample rate of 10 Hz. The NIRS probe was placed on the forehead, centered on AFpz according to the international 10-5 system and overlapping mainly the rostromedial PFC (RMPFC), a subregion commonly targeted in emotion studies (Dixon et al., 2017). The channels and placement of the emitters s1,s2,s3,s4 and detectors d1,d2,d3,d4,d5,d6,d7,d8,d9,d10 are presented in Figs. 3a and 3b.

### Signal quality check

To ensure that only good signals reached subsequent steps, we checked the raw optical data to identify outliers (< 5% or > 95% of the device's dynamic range) or excessive coefficient of variation (> 7.5%) (Orihuela-Espina et al., 2010; Zimeo Morais et al., 2017) to rule out channels with poor signal-to-noise ratio, saturation or unphysiological noise interference. By visually identifying abrupt changes aligned with accelerometer jumps, we rejected recordings degraded by motion artifacts. Three individuals participants of the group were excluded after checking, leaving a final sample of N=20.

### Signal segmentation

From the optical intensities raw data, we delimited a 390-s time interval of interest (TIOI) comprised by the five picture Pic blocks (78-s each one) sequence L . To minimize boundary and discontinuity effects (Cohen et al., 1993) of further processing methods (e.g. wavelet decomposition), we included 30-s of extra-data on both TIOI sides. Within the TIOI, all the blocks have the same length, which allows an easier subsequent comparison among them (Bendall et al., 2016). After segmentation, we obtained for each subject and wavelength, 16 raw short-signals from the short-channels plus 12 raw long-signals from the long-channels. All further processing was done by using these data segments. All the computations were done off-line with MATLAB (Version R2021b, Mathworks, Natick, MA, USA), using native functions, self-made scripts and open-source packages.

### fNIRS data preprocessing

Using functions from the MATLAB-based Homer2 NIRS package (Huppert et al., 2009), we obtained the relative changes in chromophores HbO,HbR oxy-(HbO) and deoxy-hemoglobin (HbR) concentration for each channel chA,chB,chC,chD,chE,chF,chG,chH,chl,chJ,chK,chL (Delpy et al., 1988; Kocsis et al., 2006), applying a differential path length calculated as in (Scholkmann & Wolf, 2013). HbO and HbR data were digitally low-pass filtered by using a zero-phase, 5th-order Butterworth filter, cut-off 0.2 Hz (MATLAB Signal Processing Toolbox). No high-pass filtering was used. Thus, we remove only respiratory, cardiac and high frequency instrumental components, preserving frequency bands that could overlap with hemodynamic responses (Huppert et al., 2009). As a result, for each chromophore HbO,HbR we obtained 16 time-series from short-channels plus 12 from long-channels that we called short superficial signals SS and long superficial signals LS, respectively. In this work, we focused on the long superficial signals LS and short superficial signals SS data related to the two regions-of-interest bounded by the four long-channels on the left (chA, chB,ch C, chD) and the four on the right (chl, chJ, chK, chL), as presented in Figs. 3a and 3b. We discarded the midline long-channels (chE, chF, chG, chH), avoiding signals measured through the frontal sinus (Haeussinger et al., 2011; Kurihara et al., 2012).

### Neural signal extraction

Deep fNIRS signals are contaminated by systemic and local-superficial hemodynamic changes that do not originate in the cerebral cortical layers (Kirilina et al., 2012; Saager et al., 2011; Takahashi et al., 2011). A particularly effective solution is to use multiple distance measurements (Pfeifer et al., 2018; Yücel et al., 2017) under the assumption that short-channels only record extracerebral components, whereas long-channels record both extracerebral and cerebral (Brigadoi & Cooper, 2015; Saager & Berger, 2005). On this premise, we can use short superficial signals SS data as reference superficial signal RS to remove extra-cerebral interference from long superficial signals LS and thus improve detection of the actual brain activity (Gagnon et al., 2011; Scarpa et al., 2013; Zhang et al., 2015).

Among other methods, regression can be applied assuming that physiological noise has comparable time courses in both reference superficial signals RS and long superficial signals LS, while the brain task-evoked response is independent (i.e. uncorrelated) (Maruoka et al., 2007; Saager & Berger, 2008; Zhang et al., 2015). However, previous studies have shown that superficial contamination is spatially inhomogeneous across the head (Gagnon et al., 2011; Kirilina et al., 2012) and, moreover, varies across subjects (Saager et al., 2011). Therefore, the feasibility of the RS highly depends of its recording location. To improve regression performance, it is suggested that the reference superficial signal RS be recorded as close as possible to the long superficial signal LS intended to be cleaned from surface interference (Brigadoi & Cooper, 2015; Gagnon et al., 2012).

The NIRS device allows each recorded long superficial signal LS to have three short superficial signal SS candidates that meet the proximity requirements: one obtained close to the long-channel detector (SS_{d}), one close to its source (SSₛ), and one close to its center (SS_{c}). Here, for each long superficial signal LS , we estimated the reference superficial signal RS as a combination of SS_{d} and SSₛ (RS = SS_{d} + SSₛ). This "double SS" approach has been found to be a significant improvement over the regression method (Gagnon et al., 2014). Assuming that LS contains both RS and the unknown neuronal signal (NS), it can be formulated: $NS = LS - \left({β}_{0}+{β}_{1} RS\right) ,$ where *β*₀ is the y-intercept, β₁ is the regression coefficient, RS is the reference signal, and NS the neural signal (in fact, the raw residuals). We solved linear regression by applying the MATLAB function "robustfit", which uses an iteratively reweighted least squares algorithm and is less sensitive to outliers than ordinary least-squares (Holland & Welsch, 1977). We thus obtained, for chromophores HbO,HbR 12 neural signal NS segments of equal length than reference superficial signals RS and long superficial signals LS for further processing. Finally, very slow trends were removed by subtracting a best-fit line calculated by least-squares regression. Therefore, the step of determining fNIRS related superficial signals LS,RS and neural signals NS or each of the individual of the group subject to each emotional stimuli candidate V,S,N comprises obtaining fNIRS long superficial signals LS and short superficial signals SS; and calculating a corresponding neural signal NS and reference superficial signal RS from the long superficial signals LS and the short superficial signals SS.

### Multiresolution analysis

Physiological signals are typically nonstationary and, hence, their frequency content can change over time. Wavelet transforms are especially sensitives to nonstationary features and useful for detecting hidden patterns in the raw data. Wavelet-based multiresolution analysis MRA analyze signals by decomposing them into a number of time-varying frequency components, it is oscillatory components C0,C1,C2,C3,C4,C5,C6,C7,C8,C9, providing a time-frequency representation of the data which includes both, temporal and spectral information (Jawerth & Sweldens, 1994; Mallat, 1989). The step of the method of calculating a superficial signal group response SGR and a neural signal group response NGR subject to each emotional stimuli candidate V,S,N comprises a multiresolution analysis (MRA) . Starting from the original signal and applying a progressively finer filter-bank, multiresolution analysis MRA builds a pyramidal structure of successive details (high-frequency) and approximation (low-frequency) data, up to a certain level of decomposition. In this work, we used the undecimated maximal overlap discrete transform MODWT, also known as the stationary wavelet transform (SWT) (Percival & Walden, 2000; Rhif et al., 2019), quite popular for frequency decomposition in neuroimaging research (Kajimura et al., 2023). Therefore, in this case, the multiresolution analysis MRA comprises an undecimated maximal overlap discrete transform MODWT. Since maximal overlap discrete transform MODWT is time invariant, it does not introduce ambiguities in the time domain and is best suited for synchronization analysis (Bolt et al., 2018; Kauppi et al., 2010).

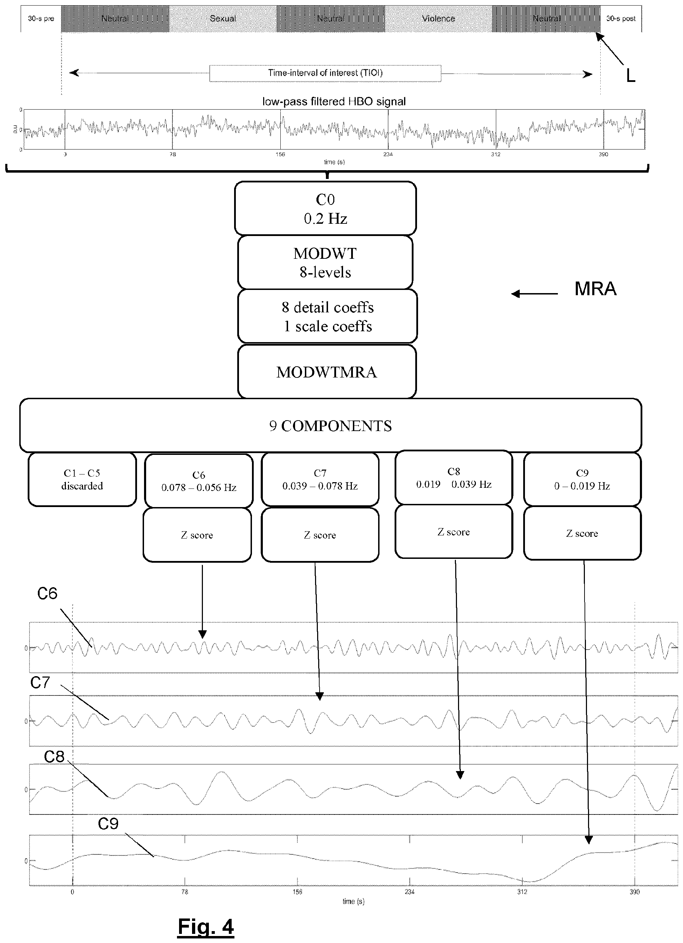

As presented in Fig. 4 the MATLAB function "modwt" was used, which is a Fourier-based implementation of the standard maximal overlap discrete transform MODWT that can manage signals of arbitrary length. As wavelet filter, we chosen the Daubechies' extremal phase wavelet with five vanishing moments (db5) (Daubechies, 1992; Mallat, 2008) to implement a 8-level decomposition. After that, we obtained eight sets of detail coefficients plus a final-level of scaling coefficients. As the coefficients are not time-aligned with the original signal we applied the MODWT output to the MATLAB function "modwtmra" to obtain a time-domain reconstruction (projection) of the signal at each decomposition level. Thus, the original signal was separated into nine oscillatory components C0,C1,C2,C3,C4,C5,C6,C7,C8,C9, here labelled as C1 to C9, corresponding to the eight detail levels plus the final approximation level and that, when added back together (element-by-element), perfectly reconstruct the signal. Since "modwtmra" behaves like a zero-phase filter, the components' features exactly line-up with the original signal because they are on the same time scale. These isolated components reflect the variability of the signal separated into nine components that, ideally, would have physical meaning and would be interpretable at their corresponding temporal scales. Therefore, the method further comprises obtaining a signal time-domain reconstruction.

As also presented in Fig. 4, as the low-pass prefiltering limited the available frequency range to 0 - 0.2 Hz, only oscillatory components C6 (0.078 - 0.156 Hz), C7 (0.039 - 0.078 Hz), C8 (0.019 - 0.039 Hz) and the approximation (smooth) component C9 (0 - 0.019 Hz) were used. Note that, for convenience, the original low-pass filtered signal is referred to here as "C0". Finally, to allow comparisons and averaging procedures the components were standardized into z-scores when necessary. Therefore, the method comprises separating a signal into oscillatory components C6,C7,C8,C9.

### Performance of regression

The efficiency of the regression to extract the neural signal NS was determined by the Pearson correlation coefficient (Erdoǧan et al., 2014) and tested for the C0 and C6-C9 signals. We computed correlation before (RS vs LS) and after regression (RS vs NS). The obtained coefficients where "gaussianized" via Fisher's transformation (Silver & Dunlap, 1987) and differences were tested using a two-tailed paired t-test. Significance thresholds were adjusted by controlling false discovery rate (FDR) at q=0.05 (Benjamini & Hochberg, 1995; Singh & Dan, 2006). The data were then back-transformed into r-statistic to simplify reporting.

### Inter-subject correlation analysis

ISC analysis relies on the assumption that the brain activity of a number of individuals is entrained to a stimulus in some common way. fMRI and fNIRS use hemodynamic signals as proxies to neural activity (Arne, 2013; Steinbrink et al., 2006) and, therefore, it can be assumed that shared fluctuations across subjects would be reflected in synchronized time-courses. On this premise, the time-course of an individual can be predicted from that of another, without the need for any prior stimulus-response model (Hasson et al., 2004). Since inferences are only based on the level of similarity of the time courses, ISC can be easily assessed by correlation (Nastase et al., 2019).

In this work, we focused on chromophore HbO because it meets both a higher sensitivity to forehead surface hemodynamics and a good correlation with fMRI BOLD in the middle frontal area across different cognitive tasks (Cui et al., 2011; Sato et al., 2013). However, other chromophores, such as HbR can also be used. By adopting the *pairwise approach* (Nastase et al., 2019), we computed the symmetric matrix of Pearson's correlation coefficients between the HbO chromophore for all combinations of subjects' pairs, within each of the five task blocks, and for each channel, oscillatory component (C0, C6-C9) and signal type (RS, LS, NS). As ISC estimator we calculated the median of the matrix's upper-triangle values, a centrality measure that is preferred to the mean because it avoids Fisher transformations and is suitable for both parametric and nonparametric testing (Chen et al., 2016). As the number of participants was 20, the median was obtained from (20² - 20)/2 = 190 values, which is good enough for ISC analysis (Pajula & Tohka, 2016).

To assess the statistical significance of the ISC estimates, we performed a nonparametric one-sample test through a subject-wise bootstrapping procedure (Chen et al., 2016), under the null hypothesis that the actual data comes randomly from an independent but identical population distribution. At each iteration, the columns and rows of the correlation matrix are replaced for those of N subjects randomly sampled with replacement. After excluding the off-diagonal 1s, the median ISC is calculated and, then, shifted by subtracting the observed median ISC (Nastase et al., 2019). We computed the approximated null distributions, over 10.000 resamples, and the observed ISC was ranked against this distribution to estimate a *p*-value. The obtained *p*-values were FDR corrected at q=0.05 (5 task blocks x 8 channels = 40 comparisons). In addition, we calculated the 95% confidence interval (CI) by bootstrapping the upper-triangle values of the observed correlation matrix (resamples = 2000).

Merely reaching ISC significance in certain oscillatory component may be misinterpreted, as could occur due to spurious correlations (more likely at lower frequencies). It is necessary to check whether the ISC is also significantly higher than in the others components. To this end, we used the test described in (Kauppi et al., 2010, 2014), which is a modified Pearson-Filon statistic based on Fisher's z-transform (ZPF) (Raghunathan et al., 1996) and recommended to test for differences between two non-overlapping dependent correlations (Krishnamoorthy & Xia, 2007). Briefly, for each pair of components (a, b) to be compared a pairwise ZPF statistics was obtained for all subject pairs in each channel. Then, a sum ZPF statistic ( ${ZPF}_{Σ}^{ab}$) for differences between two components was calculated over all subject pairs. To test for significance, an approximate permutation distribution of ${ZPF}_{Σ}^{ab}$ was generated by randomly flipping the sign of ZPFs, under the null hypothesis that they come from a distribution with zero mean (no differences). Maximal and minimal statistics over all channels were computed for each permutation to finally obtain a symmetric distribution. We generated the null distribution over 20.000 permutations and obtained critical thresholds at α = 0.05 against the largest values of the distribution, which implicitly controls the family wise error rate (FWER) for multiple comparisons (Nichols & Holmes, 2002).

Finally, to model the response patterns, we obtained the average time-course for both chromophores HbO and HbR, by channel, oscillatory component and signal type. Therefore, each of the superficial signals (LS,RS) and neural signals (NS) are associated with a chromophore (HbO,HbR), a channel and an oscillatory component. The method can comprise evaluating groups of superficial signals (LS,RS) and neural signals (NS), associated with a chromophore (HbO,HbR), a channel and an oscillatory component, obtained when individuals of the group of individuals are subject to emotional stimuli candidates V,S,N for determining one of more emotional stimuli candidates V,S,N as emotional stimuli control X, associated with the channel, chromophore and oscillatory component. Note that, though ISC was not calculated for HbR, it is recommended to contrast both chromophores to better assess fNIRS responses (Tachtsidis & Scholkmann, 2016).

### Heart rate and respiratory rate

ECG and respiratory signal were registered using a BIOPAC MP36R physiological monitoring system and the AcqKnowledge software 4.2 (Biopac Systems, Inc., Goleta, CA, USA), at a sampling rate of 500 Hz and digitally synchronized with stimuli presentation. ECG was recorded in lead II configuration with disposable electrodes, and respiratory effort by a thoracic belt transducer BIOPAC SS5LB. The raw data were post-processed with AcqKnowledge to extract the instantaneous heart rate (HR) and respiratory rate (RR). The HR and RR data were exported to MATLAB, resampled to 10 Hz using cubic spline interpolation, and then submitted to the same MRA decomposition and ISC analysis as the fNIRS data.

### Results

### Intra-session judgments and posterior SAM ratings

Pooling the judgments of all participants, we found that violent pictures were perceived as unpleasant in 97% of the cases. Sexual pictures were judged rather inconsistently, 43% pleasant, 24% unpleasant and 33% indifferent. Neutral ones were rated 72% as indifferent and the rest as pleasant. Regarding SAM ratings, ANOVA test revealed significant effects for valence (*F* (2, 38) = 62.24, ip *<* 0.001) and arousal (*F* (2, 38) = 66.99, *p* < 0.001). Post-hoc analysis showed higher valence ratings for sexual (M=7.40, SD=1.46) than for violence (M=2.30, SD=0.69). The arousal did not differ between sexual (M=7.77, SD=1.49) and violence (M=8.40, SD=0.73). Neutral pictures were rated significantly different for both valence (M=5.17, SD=1.75) and arousal (M=3.95, SD=1.66). These results were consistent with normative values, confirming that participants' emotional states were induced as expected.

### Regression performance

A summary statistic of r-values before (LS vs RS) and after regression (NS vs RS) was obtained. As expected, long superficial signals LS were strongly correlated with reference superficial signals RS on all channels and time scales, reflecting a clear contamination of surface hemodynamics. The regression procedure successfully denoised long superficial signals LS and provided a reasonably clean neural signals NS, as evidenced by the drastic reduction in correlation. All contrasts (two-tailed paired t-test) yielded significant differences (*p* < 0.05, FDR-corrected). Both long superficial signal LS and reference superficial signal RS give a superficial signal group response SGR and a the neural signal NS give the neural signal group response NGR.

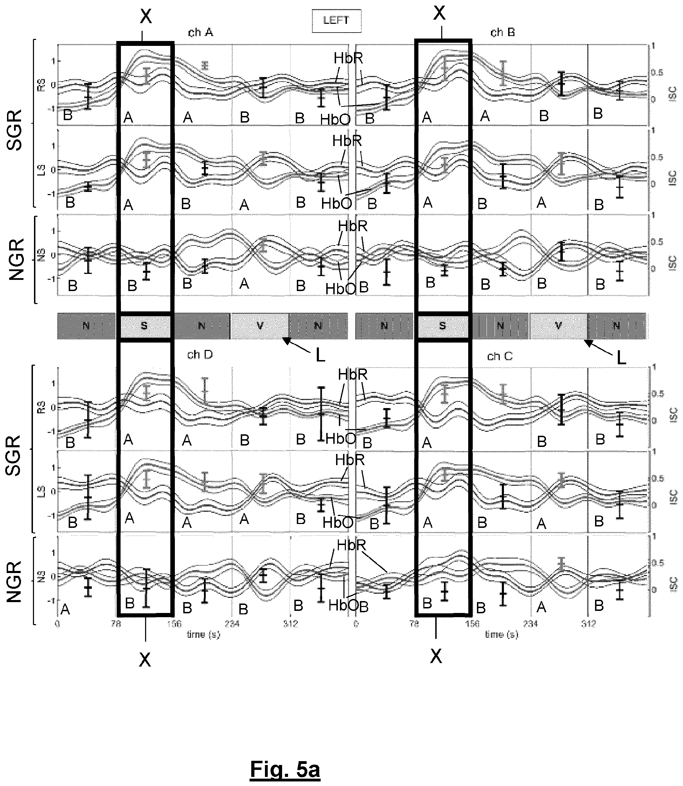

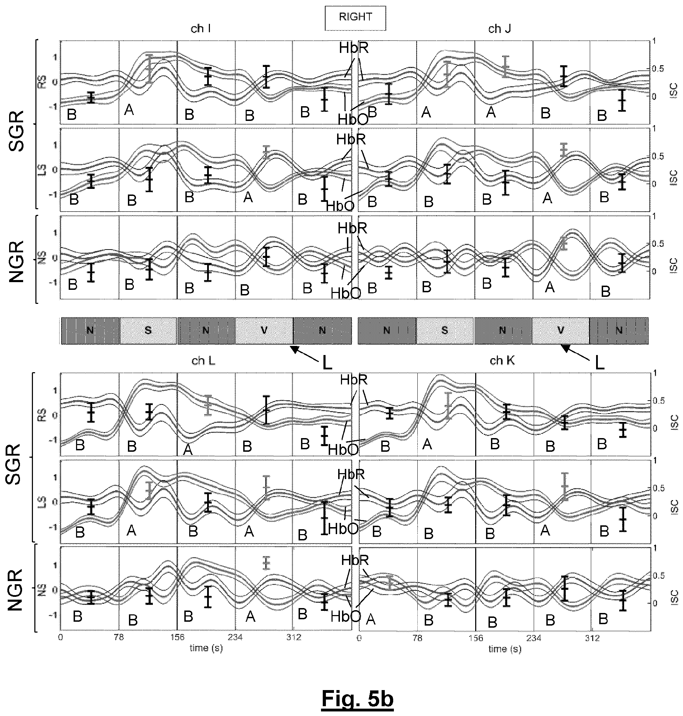

Figs. 5a and 5b presents group average of the z-scored time courses of chromophores HbO and HbR in C9. Note that in all presented cases levels of HbO start and finish below levels of HbR. Thick and thin curves depict the mean and the standard error of mean, respectively. Fig. 5a presents RS, LS (superficial signal group response SGR) and NS (neural signal group response NGR) for channels chA, chB, chC and chD (left to right columns) and Fig. 5b for channels chl, chJ, chK and chL. Thin vertical lines mark the onset of each picture block, in correspondence with the sequence L of emotional stimuli candidate V,S,N rectangles drawn in the middle of the graph (N = neutral; S = sexual; V = violence). Within each block, the median ISC and CI (95%) for HbO are plotted and indicated, per each block, as with no-significance B (no significant) or with significance A (significant; p < 0.05, FDR-corrected). Although HbR ISCs were not used, time courses of HbR are plotted for comparison purposes.

### ISC in NS

No significant ISC was found in any channel or block in components C6 to C8 nor in the original C0 signal (data not shown). However, violence pictures induced strong ISCs in C9 of channels A, C, J and L, reaching significance (p *<* 0.05, FDR corrected), with median values in the range 0.43 to 0.7 (NS in Figs. 5a, 5b). Therefore a threshold of significance was established for p = 0.05, lower values considered no-significance B and upper or equal values considered significance A.

The ZPF test confirmed that ISC values were significantly higher in these channels for C9 than for any other component (p < 0.05, FWER corrected). Of note, for the rest of channels the ZPF test was also significant. Therefore, ISC concentrates in the negative valence at the lowest frequency band. Figure 5a and 5b, NS shows the average time courses of the z-scored HbO and HbR in C9, where a pattern of initial HbO decrease and HbR increase followed by a change in the opposite direction can be observed. This U-shaped HbO pattern explains the strong ISCs found in this block.

### ISC in RS

Again, no significant ISC was found in components C6 to C8 and C0 was also not significant (data not shown). However, sexual pictures triggered consistent responses in C9, even extending to the subsequent neutral block (LS in Figs. 5a, 5b). This effect was present in all channels except L, with median ISC values ranging from 0.39 to 0.56, and in the form of a common pattern of a sharp increase in HbO along with an initial decrease in HbR followed by an increase. The ZPF test showed that ISC values were also significantly higher in these channels for C9 (p < 0.05, FWER corrected). The ZPF test was also significant for channel L. Interestingly, violence pictures did not drive coherent responses in RS.

### ISC in LS

Figs. 5a and 5b present that only in C9 were significance A ISCs found in five channels for the sexual block (0.33 to 0.57) and in all the channels for the violence block (0.36 to 0.6). The LS time courses look like a combination of RS and NS (especially evident in the sexual block), suggesting a mixture of shallow and deep components. This predictable finding underscores the susceptibility of forehead recordings to interference from shallow hemodynamics.

Therefore, significance A,B of the group response for each emotional stimuli candidate V,S,N is calculated using significance threshold p of an intersubject correlation. This threshold of significance is established for p = 0.05, lower values considered no-significance B and upper or equal values considered significance A.

### ISC in HR and RR

Interestingly, no significant ISC was detected in any component (not even in C9) for either heart rate (HR) or respiratory rate (RR). However, in C9 their time courses seem to fluctuate to some extent influenced by emotional content, but never reach ISC significance. This finding suggests that the consistent responses seen in HbO signals are not explained by these systemic activities, likely due to more idiosyncratic responses for HR and RR.

### Discussion

The present work aimed to investigate whether the presentation of a well-structured sequence of affective pictures would induce emotional states long enough to allow the detection of shared responses from multi-distance fNIRS recordings in the forehead. We applied MRA for frequency decomposition of the fNIRS signals into narrowband oscillatory components, which were in turn subjected to ISC analysis. We found coherent HbO patterns in a sample of healthy young women, which were confined to the lowest frequency band (0 - 0.019 Hz) and were specifically triggered in shallow signals by sexual content (high-arousal, positive-valence) and in deep signals by violence (high-arousal, negative-valence). Overall, our findings support the view that negative stimuli would more consistently synchronize PFC activity across individuals, likely recruiting common cerebral circuits relevant to survival, whereas positive stimuli would elicit rather idiosyncratic responses (Nummenmaa et al., 2012). Specifically, they also support that women show a strong engagement of the medial PFC with anger and fear stimuli (Li et al., 2020; Stevens & Hamann, 2012; Whittle et al., 2011), whereas sexual imagery does not significantly recruit it (Cyders et al., 2016) but instead elicits consistent responses in surface hemodynamics. On another hand, they further highlight the importance of controlling surface activity and identifying shared fNIRS responses on the most informative time scales, in both shallow and denoised deep signals. The following discussion is not intended to address the underlying neural mechanisms, but to contrast our results with those of similar studies on emotion.

### Superficial and deep separation

Long superficial signal LS was strongly correlated with reference superficial signal RS in all channels and components (see Figs. 5a,5b), which corroborates that surface activity is a major source of interference (Kirilina et al., 2012; Tachtsidis & Scholkmann, 2016; Yamada et al., 2012). However, the correlation was greatly reduced for the neural signal NS components extracted by multi-distance regression. In oscillatory component C9, violence pictures induced consistent ISCs in both long superficial signal LS (all channels) and neural signal NS (four channels), but not in reference superficial signal RS, suggesting a deep origin of HbO changes (see Figs. 5a,5b). It is likely that regression over-attenuated the responses of some individuals and resulted in fewer significant NS channels, which would be supported by the ZPF test.

In contrast, sexual scenes promoted significant ISCs in virtually all reference superficial signals RS and long superficial signals LS channels, both considered to be superficial signal group response SGR, but none in neural signal NS, considered to be the neural signal group response NGR, thus pointing to a surface origin. Although unlikely, it would not be fully excluded that concurrent, highly correlated, superficial and truly deep activity resulted in neural signal NS suppression by the regression itself. In any case, the sex block proved that the regression-based cleaning procedure was quite effective, as seen by its response by the individuals of the group in oscillatory component C9, chromophore HbO, and channels chA, chB, chC, chD of Figs. 5a and 5b. In fact, the detected emotional stimuli candidate V,S,N, sexual S has an identifiable effect on surface activity can be advantageously used as a emotional stimuli control X to check the reliability of signal denoising methods as known by the skilled person.

Therefore, following this methodology, a computer implemented method for selecting emotional stimuli control X among emotional stimuli candidates V,S,N for a group of individuals can be followed by a computer, to be used in signal denoising to isolate the true cerebral signal, comprising the method the sequence of steps determining fNIRS related superficial signals LS,RS and neural signals NS for each of the individual of the group subject to emotional stimuli candidates; calculating a superficial signal group response SGR and a neural signal group response NGR for each emotional stimuli candidate, and the significance A,B of each group responses; and selecting as an emotional stimuli control X for the group an emotional stimuli-that does have a significance A superficial signal group response SGR, and has a no-significance B corresponding neural signal group response NGR.

In the previous example, an emotional stimuli candidate S, sexual, was considered to be an emotional stimuli control X, as it presents a significance A superficial signal group response SGR, and a no-significance B corresponding neural signal group response NGR, for chromophore HbO, for any of channels chA, chB,chC,chD in oscillatory component C9. Therefore this emotional stimuli control X can be used later in signal denoising to isolate the true cerebral signal. However, other emotional stimuli control X can be decided for a different group of individuals, for which the method has to be done again.

## Claims

1. Computer implemented method for selecting emotional stimuli control (X) among emotional stimuli candidates (V,S,N) for a group of individuals to be used in signal denoising to isolate the true cerebral signal, wherein the method comprises the following sequence of steps:
- determining fNIRS related superficial signals (LS,RS) and neural signals (NS) for each of the individual of the group subject to emotional stimuli candidates;
- calculating a superficial signal group response (SGR) and a neural signal group response (NGR) for each emotional stimuli candidate, and the significance (A,B) of each group responses; and
- selecting as an emotional stimuli control (X) for the group an emotional stimuli-that does have a significance (A) superficial signal group response (SGR), and has a no-significance (B) neural signal group response (NGR).

2. The method according to the preceding claim **characterized in that** the step of determining fNIRS related superficial signals (LS,RS) and neural signals (NS) or each of the individual of the group subject to each emotional stimuli candidate (V,S,N) comprises:
- obtaining fNIRS long superficial signals (LS) and short superficial signals (SS); and
- calculating a corresponding neural signal (NS) and reference superficial signal (RS) from the long superficial signals (LS) and the short superficial signals (SS).

3. The method according to any of the preceding claim **characterized in that** emotional stimuli candidates (V,S,N) are associated with threshold levels of valence and arousal.

4. The method according to any of the preceding claims **characterized in that** the step of determining fNIRS superficial signal (LS,RS) and neural signals (NS) for each of the individual of the group subject to each emotional stimuli candidate (V,S,N) comprises subjecting each individual to a sequence (L) of emotional stimuli candidates.

5. The method according to any of the preceding claims, **characterized in that** emotional stimuli candidates (V,S,N) are represented by pictures (Pic) to be seen by the individuals.

6. The method according to any of the preceding claims, **characterized in that** superficial signals (LS,RS) and neural signals (NS) are associated with a chromophore (HbO,HbR), a channel (chA,...,chl) and an oscillatory component (C0,...C9).

7. The method according to any of the preceding claims, **characterized in that** the significance (A,B) of the group response for each emotional stimuli candidate (V,S,N) is calculated using significance threshold of an intersubject correlation.

8. The method according to any of the preceding claims, **characterized in that** the step of calculating a superficial signal group response (SGR) and a neural signal group response (NGR) subject to each emotional stimuli candidate (V,S,N) comprises a multiresolution analysis (MRA) .

9. The method according to the preceding claim, **characterized in that** the multiresolution analysis (MRA) comprises an undecimated maximal overlap discrete transform (MODWT).

10. The method according to the preceding claim, **characterized in that** it further comprises obtaining a signal time-domain reconstruction.

11. The method according to the preceding claim, **characterized in that** the signal time-domain reconstruction comprises separating a signal into oscillatory components (C0,...,C9).

12. A data processing apparatus comprising a processor configured to perform the steps of the method of any of claims 1 to 12.

13. A computer program comprising instructions which, when the program is executed by a computer, cause the computer to carry out the steps of the method of any of claims 1 to 12.

14. A computer-readable storage medium comprising instructions which, when executed by a computer, cause the computer to carry out the steps of the method of any of claims 1 to 12.
